(19) Europäisches Patentamt
European Patent Office
Office européen des brevets

(11) Veröffentlichungsnummer : **0 100 862**
**B1**

(12) # EUROPÄISCHE PATENTSCHRIFT

(45) Veröffentlichungstag der Patentschrift : **19.08.87**

(51) Int. Cl.⁴ : **C 07 D321/06**, C 08 F 2/50, C 08 F299/04

(21) Anmeldenummer : **83106533.9**

(22) Anmeldetag : **05.07.83**

(54) **2-Acylierte Dihydro-1,3-dioxepine, Verfahren zu ihrer Herstellung und ihre Verwendung als Photoinitiatoren.**

(30) Priorität : **16.07.82 DE 3226617**

(43) Veröffentlichungstag der Anmeldung : **22.02.84 Patentblatt 84/08**

(45) Bekanntmachung des Hinweises auf die Patenterteilung : **19.08.87 Patentblatt 87/34**

(84) Benannte Vertragsstaaten : **AT BE CH DE FR GB IT LI NL**

(56) Entgegenhaltungen : **Keine Entgegenhaltungen**

(73) Patentinhaber : **CIBA-GEIGY AG**
**Klybeckstrasse 141**
**CH-4002 Basel (CH)**

(72) Erfinder : **Scharf, Hans-Dieter, Prof. Dr.**
**Greppstrasse 15 a**
**D-5106 Roetgen (DE)**
Erfinder : **Frauenrath, Herbert, Dr.**
**Haarhofstrasse 72**
**D-5100 Aachen (DE)**
Erfinder : **Heine, Hans-Georg, Dr.**
**Am Heckerhof 14**
**D-4150 Krefeld 1 (DE)**
Erfinder : **Rudolph, Hans, Dr.**
**Haydnstrasse 9**
**D-4150 Krefeld 1 (DE)**
Erfinder : **Neuhaus, Karl-Friedrich, Dr.**
**Bodelschwinghstrasse 14**
**D-4150 Krefeld 1 (DE)**
Erfinder : **Bendszus, Otto**
**Breite Strasse 92**
**D-4150 Krefeld 1 (DE)**

**EP 0 100 862 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft in 2-Stellung acylierte Dihydro-1.3-dioxepine, ein Verfahren zu ihrer Herstellung durch Umsetzung von 2-Buten-1,4-diol mit einem Benzil oder mit einem Benzilmonoketal sowie die Verwendung dieser Dihydro-1,3-dioxepine als Photoinitiatoren.

Die Verwendung von lichtempfindlichen Verbindungen, die bei Bestrahlung Radikale bilden, als Photoinitiatoren ist lange bekannt (Übersicht : J. Kosar, Light-Sensitive Systems, Wiley, New York 1965). Einer der Photoinitiatoren, die auch Eingang in den Markt gefunden haben, ist Benzildimethylketal. Seine hohe Reaktivität wird sehr geschätzt ; nachteilig wird empfunden, daß damit hergestellte Überzüge unter Lichteinfluß vergilben.

Aufgabe der Erfindung war es daher, vergilbungsärmere Photoinitiatoren bereitzustellen, die die Reaktivität des Benzildimethylketals mindestens erreichen, möglichst aber übertreffen.

Überraschenderweise wurde gefunden, daß 2-acylierte Dihydro-1,3-dioxepine der Formeln

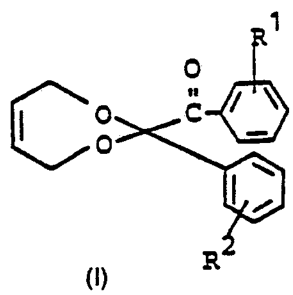

(I)

und

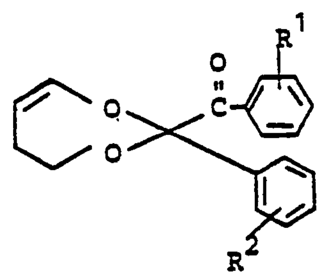

(II)

hochreaktive Initiatoren sind, die die Herstellung photogehärteter Überzüge mit wesentlich verminderter Vergilbungsneigung zulassen.

Die erfindungsgemäßen Verbindungen I lassen sich durch Umsetzung von cis-2-Buten-1,4-diol mit Benzilen der Formel

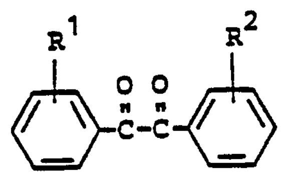

(III)

oder mit Benzilmonoketalen der Formel

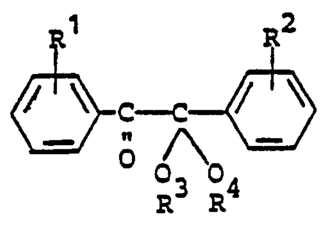

(IV)

herstellen, wobei

$R^1$ und $R^2$ unabhängig voneinander ein Wasserstoffatom, einen $C_1$-$C_4$-Alkoxy- oder Alkylrest oder ein Halogenatom und

$R^3$ und $R^4$ unabhängig voneinander einen $C_1$-$C_4$-Alkylrest bedeuten.

Die Reaktion wird in Gegenwart eines sauren Katalysators, insbesondere aber in Gegenwart von Thionylchlorid und einem Verdünnungsmittel, durchgeführt.

Die erfindungsgemäßen Verbindungen II lassen sich durch Isomerisierung aus den Verbindungen I herstellen. Als Isomerisierungskatalysatoren können starke Basen oder Übergangsmetallhydride verwendet werden.

Es ist zwar bekannt, aromatische 1,2-Diketone mit primären einwertigen Alkoholen in Gegenwart eines Derivats der schwefligen Säure, z. B. Dialkylsulfit, Thionylchlorid, zu den entsprechenden Monoketalen umzusetzen (DE-AS 2 337 813) ; eine technisch brauchbare Einstufensynthese cyclischer Monoketale aromatischer 1,2-Diketone ist unseres Wissens bisher nicht bekannt gewesen. Das erfindungsgemäße Herstellungsverfahren ist umso überraschender als beispielsweise Benzil protonenkatalysiert selbst mit geringen Mengen Ethylenglykol zum Bisketal reagiert (Rec. Trav. Chim. 57, 133 (1938)). Überraschend ist aber auch, daß unter den Reaktionsbedingungen das cis-2-Buten-1,4-diol nicht nennenswert dehydratisiert wird (vgl. Compt. rend. 223, 907 (1946) ; J. Amer. Chem. Soc. 54, 4385 (1932)). Bei der Ketalisierung in Gegenwart von Thionylchlorid erscheint überraschend, daß keine Umsetzung des Thionylchlorids mit dem 2-Buten-1,4-diol zu beobachten ist (vgl. z. B. J. Org. Chem. 39, 848 (1974) ; Bull. Inst. Chem. Res., Kyoto Univ. 1971, 49 (3), 179-99).

Gegenstand der Erfindung sind also Verbindungen der Formel

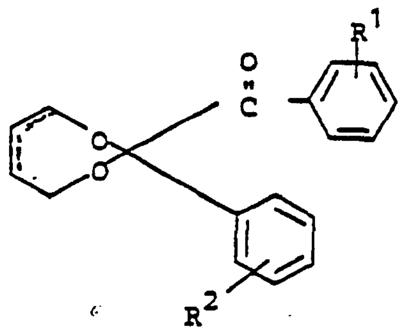

(V)

worin $R^1$ und $R^2$ unabhängig voneinander ein Wasserstoffatom, einen $C_1$-$C_4$-Alkoxy- oder Alkylrest, vorzugsweise einen Methylrest, oder ein Halogenatom, vorzugsweise ein Chloratom, bedeuten.

Weiterer Gegenstand der Erfindung ist ein Verfahren zur Herstellung der Verbindungen V durch Umsetzung von cis-2-Buten-1,4-diol mit einem Benzil der Formel

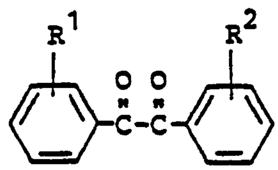

(III)

oder mit einem Benzilmonoketal der Formel

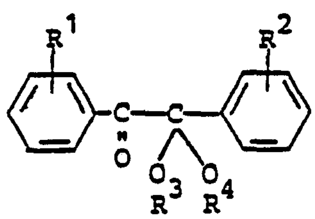

(IV)

wobei

$R^1$ und $R^2$ die oben angegebene Bedeutung besitzen und

$R^3$ und $R^4$ unabhängig voneinander einen $C_1$-$C_4$-Alkylrest bedeuten, in Gegenwart eines sauren Katalysators und gegebenenfalls anschließende Isomerisierung.

Weiterer Gegenstand der Erfindung ist die Verwendung der Verbindungen V als Photoinitiatoren, vorzugsweise im UV-Bereich von 200-500 nm, insbesondere 300-450 nm.

Bevorzugte 1,2-Diketone III sind Benzil, 4,4'-Dimethylbenzil, 4,4'-Dichlorbenzil, 4-Methylbenzil, 3-Methoxybenzil, 4-Chlorbenzil.

Bevorzugte Monoketale IV sind Benzildimethylketal, Benzildiethylketal, Benzildipropylketal, 4,4'-Dimethylbenzildimethylketal, 4,4'-Dichlorbenzildimethylketal und 4-Chlorbenzildimethylketal. Da Ethanol und vor allem Methanol besonders leicht aus dem Reaktionsgemisch entfernt werden können, empfiehlt es sich, die jeweiligen Dimethyl- oder Diethylketale einzusetzen.

Das erfindungsgemäße Verfahren kann in einem Verdünnungsmittel, das gegebenenfalls gleichzeitig als Schleppmittel für das entstehende Reaktionswasser dient, durchgeführt werden. Bevorzugte Verdünnungsmittel sind z. B. Benzol, Toluol, Methanol, Ethanol, Isopropanol, Isobutanol und überschüssiges cis-2-Buten-1,4-diol. Wird cis-Buten-1,4-diol als Verdünnungsmittel verwendet, so empfiehlt sich, zur Entfernung flüchtiger Reaktionsprodukte (Wasser, Alkohole) aus der Reaktionsmischung die Reaktion unter vermindertem Druck auszuführen.

Zur Durchführung des erfindungsgemäßen Verfahrens vereinigt man das Benzil III bzw. das Monoketal IV mit cis-2-Buten-1,4-diol, gegebenenfalls unter Zusatz eines Verdünnungsmittels und/oder eines Katalysators.

Als Katalysatoren für das erfindungsgemäße Verfahren ausgehend vom Benzil III eignen sich vor allen Thionylchlorid und p-Toluolsulfonsäure, ausgehend vom Monoketal IV vor allem Schwefelsäure, p-Toluolsulfonsäure, Phosphorsäure, Chlorwasserstoff, Bortrifluoridetherat, Thionylchlorid und saure Ionenaustauscher (vgl. « Methoden der Organischen Chemie » (Houben-Weyl), Bd. VI/3, S. 203 ff., insbesondere S. 207 und 215, Georg Thieme Verlag, Stuttgart 1965). Die Katalysatoren werden in der Regel in Mengen von 0,1 bis 5 Gew.-%, bezogen auf Ausgangsprodukt III bzw. IV, eingesetzt.

Während man die mit Thionylchlorid katalysierten Reaktionen in der Regel bei — 20 bis + 20 °C durchführt, werden die mit anderen Katalysatoren beschleunigten Reaktionen im allgemeinen durch Erwärmen auf Temperaturen von 40 bis 110, vorzugsweise von 50 bis 80, durchgeführt. Der Fortgang der Reaktion kann dünnschichtchromatographisch verfolgt werden. In der Regel wird man die Reaktionsmischung neutralisieren und bei Verwendung eines wasserunlöslichen Verdünnungsmittels dieses abziehen, im Falle eines wasserlöslichen Verdünnungsmittels das Reaktionsprodukt mit Wasser ausfällen. Falls das erhaltene Rohprodukt weiter gereinigt werden soll, kann man eine Destillation oder Umkristallisation anschließen.

Zur Isomerisierung der 4,7-Dihydro-1,3-dioxepine I zu 4,5-Dihydro-1,3-dioxepinen II kann man gemäß Chem. Ber. 113, 1472 (1980) oder nach H. Suzuki et al., Tetrahedron Letters 1980, 4927, verfahren. Bevorzugte Isomerisierungskatalysatoren sind starke Basen, wie z. B. Kalium-tert.-butylat, und Übergangsmetallhydride, wie z. B. Rutheniumhydrid.

Beispiele für Verbindungen der Formel V sind :

2-Benzoyl-2-phenyl-4,7-dihydro-1,3-dioxepin,
2-Benzoyl-2-phenyl-4,5-dihydro-1,3-dioxepin,
2-(4-Methylbenzoyl)-2-(4-methylphenyl)-4,7-dihydro-1,3-dioxepin,
2-(4-Chlorbenzoyl)-2(4-chlorphenyl)-4,7-dihydro-1,3-dioxepin.

Als Substanzen, deren Polymerisation durch die erfindungsgemäßen Verbindungen V initiiert werden kann, kommen alle radikalisch polymerisierbaren Verbindungen oder Gemische in Betracht, z. B. also Olefine wie Ethylen, konjugierte Diene wie Butadien, Isopren, Chloropren ; Vinylchlorid, Vinylidenchlorid ; aromatische Vinylverbindungen wie Styrol, Divinylbenzol ; Vinylester, insbesondere Vinylacetat und Vinylpropionat ; Vinylether wie Vinylpropylether, Vinylisobutylether ; Acrylsäure und Methacrylsäure und deren Derivate wie Ester, insbesondere mit aliphatischen Alkoholen mit 1 bis 5 C-Atomen, Nitrile, Amide usw. ; Di-(vinylphenyl)carbonate ; Diallylphthalat, Diallylcarbonat, Diallylfumarat ; Di-(allylphenyl)carbonate ; Polyol-poly(meth)acrylate ; N,N'-Methylen-bis-(meth)acrylamid.

Besonders bevorzugte zu polymerisierende Substanzen sind (meth)acryloylgruppenhaltige Präpolymere, wie z. B. Polyester(meth)acrylat, Epoxy(meth)acrylat, Urethan(meth)-acrylat sowie ungesättigte Polyester, die gegebenenfalls mit Reaktivverdünnern, wie z. B. (Meth)Acrylsäureestern oder Styrol verdünnt sein können. Hierbei umfaßt der Begriff « (Meth)Acrylat » sowohl Acrylsäurederivate, Methacrylsäurederivate als auch Mischungen dieser Komponenten.

Bevorzugte α,β-ethylenisch ungesättigte Polyester sind die üblichen Polykondensationsprodukte mindestens einer α,β-ethylenisch ungesättigten Dicarbonsäure mit in der Regel 4 oder 5 C-Atomen oder deren ersterbildenden Derivate, z. B. ihren Anhydriden, gegebenenfalls in Abmischung mit bis zu 200 Mol-%, bezogen auf die ungesättigten Säurekomponenten, mindestens einer aliphatischen gesättigten Dicarbonsäure mit 4 bis 10 C-Atomen oder einer cycloaliphatischen oder aromatischen Dicarbonsäure mit 8-10 C-Atomen oder deren esterbildenden Derivate mit mindestens einer Polyhydroxyverbindung, insbesondere Dihydroxyverbindung, mit 2 bis 8 C-Atomen — also Polyester, wie sie bei J. Björksten et al., « Polyesters and their Applications », Reinhold Publishing Corp., New York 1956, beschrieben sind.

Weitere Systeme, die mit den erfindungsgemäßen Verbindungen V gehärtet werden können, sind z. B. in den DE-OS 2 737 406 und 2 841 880 beschrieben.

## Beispiele

A 1. Herstellung der Bindemittel

1. Herstellung eines Urethanacrylats

1 065.6 g Isophorondiisocyanat, 0,11 g Zinnoctoat und 0,53 g 2,5-Di-tert.-butylhydrochinon wurden auf 50-60 °C erwärmt und unter Rühren und Überleiten von trockener Luft 445,4 g Hydroxyethylacrylat zugetropft. Nach Erreichen eines NCO-Wertes von 16,1 Gew.-% wurden 3,1 g Zinnoctoat hinzugefügt und unter Beibehaltung der Temperatur 74,88 g Thiodiglykol und 1 080 g eines ethoxylierten Trimethylolpropans mit einer OH-Zahl von 250 zugesetzt. Der Ansatz wurde mit 1 120 g Hexandiol-1,6-bisacrylat gemischt und solange bei 65 °C gerührt, bis der NCO-Wert unter 0,1 Gew.-% sank (Bindemittel 1).

2. Herstellung eines ungesättigten Polyesterharzes

Aus 2 157 g Maleinsäureanhydrid, 451 g Propylenglykol, 1 441 g Diethylenglykol, 941,3 g Trimethylolpropandiallylether und 428,3 g Diethylenglykolmonobutylether wurde durch Schmelzkondensation ein Polyester mit einer Säurezahl von 15-20 hergestellt. Der erhaltene Polyester wurde mit Styrol zu einer 70 gew.-%igen Lösung verdünnt (Bindemittel 2).

A 2. Herstellung des Reaktivverdünners

I) Herstellung von oxethyliertem Trimethylolpropan mit einem Oxethylierungsgrad von 3,75

402 g Trimethylolpropan wurden in einem Kolben, der mit Gaseinleitungsrohr, Rührer und Thermometer ausgestattet war, mit 1 g Natriummethylat versetzt und auf 100 °C erwärmt. Es wurden langsam 495 g Ethylenoxid eingeleitet. Nach Beendigung der Reaktion wurde der Kolben kurzzeitig evakuiert und dann abgekühlt. Es resultierte ein farbloses Produkt mit einer OH-Zahl von 550 und einer Viskosität von 500 cP bei 20 °C im Höppler-Viskosimeter.

Wie durch Gaschromatographie bestimmt wurde, besaß das Produkt folgende Zusammensetzung :

4 Gew.-% Monooxethylierungsprodukt,
14 Gew.-% Dioxethylierungsprodukt,
29 Gew.-% Trioxethylierungsprodukt,
30 Gew.-% Tetraoxethylierungsprodukt,
16 Gew.-% Pentaoxethylierungsprodukt und
6 Gew.-% Hexaoxethylierungsprodukt.

II) Herstellung des Triacrylats des nach I) erhaltenen oxethylierten Trimethylolpropans

300 g des nach I) erhaltenen oxethylierten Trimethylolpropans mit einem Oxethylierungsgrad von 3,75 wurden in 50 ml Toluol mit 216 g Acrylsäure in Gegenwart von 2,5 g p-Toluolsulfonsäure und 0,2 g Toluhydrochinon azeotrop verestert, bis kein Wasser mehr isoliert werden konnte. Nach Beendigung der Reaktion wurde mit der äquivalenten Menge Soda, bezogen auf p-Toluolsulfonsäure, neutralisiert, das Toluol im Vakuum abdestilliert (Enddruck 1 Torr, Endtemperatur des Reaktionsgutes 100 °C) und das Produkt filtriert. Es resultierte eine hellgelbe geruchlose Flüssigkeit mit einer Viskosität von 102 cP (20 °C Höppler Viskosimeter), einem Brechungsindex $n_D^{20} = 1.4738$, einer OH-Zahl von 13 und Säurezahl 0,8. Das erhaltene Produkt wurde als Reaktivverdünner für Beispiel 6 eingesetzt.

B. Herstellung der erfindungsgemäßen Verbindungen I

Beispiel 1

Zu einer Suspension von 77 g (0,3 Mol) Benzildimethylketal in 160 g (~ 1,8 Mol) cis-2-Buten-1,4-Diol gab man 1 g p-Toluolsulfonsäure und erwärmte unter einem Druck von 10-15 Torr auf 50-60 °C. Nach 45 Minuten erhielt man eine klare Lösung, deren Temperatur auf 65-70 °C anstieg. Nach weiteren 45 Minuten war die Reaktionsmischung zu einem Kristallbrei erstarrt. Nach insgesamt 4 Stunden kristallisierte man aus Isopropanol um und erhielt 80 g (96 %) 2-Benzoyl-2-phenyl-4,7-dihydro-1,3-dioxepin als farblose Kristalle vom Schmp. 118-119 °C.

Beispiel 2

Eine Mischung aus 21 g (0,1 mol) Benzil, 53,5 g (~ 0,6 Mol) cis-2-Buten-1,4-diol und 0,5 g p-Toluolsulfonsäure wurde bei 50-60 °C und einem Druck von 10-13 Torr 9 Stunden lang gerührt. Das Reaktionsprodukt löste man in Dichlormethan, wusch mit Wasser bis zur Neutralreaktion, trocknete die Dichlormethanphase über wasserfreiem Natriumsulfat und erhielt nach Eindampfen der filtrierten Lösung 26,7 g gelbgefärbte Kristalle, die lt. NMR-Analyse zu 70 % aus 2-Benzoyl-2-phenyl-4,7-dihydro-1,3-dioxepin bestanden. Fraktionierende Destillation und Kristallisieren der bei 162 bis 168 °C/0,4 Torr siedenden Fraktion lieferten 9,9 g farbloses 2-Benzoyl-2-phenyl-4,7-dihydro-1,3-dioxepin vom Schmp. 118-119 °C.

Beispiel 3

Zu einer Suspension von 105 g (0,5 Mol) Benzil in 107,1 g (0,9 Mol) Thionylchlorid gab man unter Rühren und Kühlen auf eine Temperatur unter 10 °C 60,8 g (1,9 Mol) Methanol. Man rührte zunächst bis zum Erreichen der Raumtemperatur, dann 2 Stunden bei 50 °C. Zu der klaren, schwach gelb gefärbten Lösung gab man 52,8 g (0,6 Mol) cis-2-Buten-1,4-diol und destillierte flüchtige Produkte bei 10 Torr und 50 °C ab. Man rührte 3 Stunden bei 60-65 °C unter Normaldruck nach, setzte 15 g Kaliumcarbonat und 200 ml Isopropanol zu und fällte das Reaktionsprodukt durch Zutropfen von 100 ml Wasser. Man erhielt 110,4 g 2-Benzoyl-2-phenyl-4,7-dihydro-1,3-dioxepin vom Schmp. 115-119 °C. Nach Umkristallisieren aus Isopropanol betrug der Schmp. 118-119 °C.

Beispiel 4

Zu einer Suspension von 105 g (0,5 Mol) Benzil in 119 g (1 Mol) Thionylchlorid tropfte man bei 0-5 °C die Lösung von 52,8 g (0,6 Mol) cis-2-Buten-1,4-diol in 36 g Isopropanol. Man ließ die Reaktionsmischung sich auf Raumtemperatur erwärmen, rührte darauf 3 Stunden bei 50 °C, destillierte bei 10 Torr/50 °C flüchtige Reaktionsprodukte ab und hielt anschließend die Reaktionsmischung 2 Stunden auf einer Temperatur von 50-60 °C. Nach Zugabe von 10 g Kaliumcarbonat und 200 ml Isopropanol fällte man das Reaktionsprodukt durch Eintropfen von 100 ml Wasser. Man erhielt 95,3 g ockerfarbene Kristalle, die aus Isopropanol umkristallisiert wurden ; Schmp. 112-116 °C.

C. Herstellung einer erfindungsgemäßen Verbindung II

Beispiel 5

Unter Stickstoffatmosphäre wurden 500 mg $H_2Ru$ $[P(C_6H_5)_3]_4$ zu 15 g 2-Benzoyl-2-phenyl-4,7-dihydro-1,3-dioxepin gegeben. Die Reaktionsmischung wurde bei 140 °C gehalten und gerührt. Nach 2 Stunden wurden weitere 250 mg $H_2Ru[P(C_6H_5)_3]_4$ zugegeben, und das Rühren wurde 2 Stunden fortgesetzt. Nach dem Abkühlen auf 20 °C wurden 200 ml Ether zugegeben. Die Etherschicht wurde zweimal mit je 100 ml Wasser gewaschen und über Kaliumcarbonat getrocknet. Nach dem Filtrieren wurden 2,0 g Aktivkohle zugesetzt, und die Lösung wurde 1/2 Stunde am Rückfluß gekocht. Nach erneutem Filtrieren wurde die Lösung auf ca. 100 ml eingeengt und 50 ml Petrolether zugegeben. Beim Abkühlen bildeten sich farblose Kristalle von 2-Benzoyl-2-phenyl-4,5-dihydro-1,3-dioxepin vom Schmp. 78-79 °C.

D. Verwendung der erfindungsgemäßen Verbindungen

Die nachfolgend angegebenen Teile sind Gewichtsteile.

Beispiel 6

Ein strahlenhärtbarer Lack, bestehend aus 150 Teilen Bindemittel 1, 112,5 Teilen Hexandiol-1,6-bisacrylat, 77,5 Teilen Reaktivverdünner nach A 2 II und 8,5 Teilen der in Tabelle 1 angegebenen Photoinitiatoren wurde mittels eines drahtumwickelten Metallstabes zu einem 8 µm dicken Film auf Kunstdruckpapier aufgezogen.

Die beschichteten Proben wurden anschließend auf einem regelbaren Transportband unter einem Hanovia-UV-Strahler (80 W/cm, Abstand 8 cm) durchgeführt. In Tabelle 1 sind die Geschwindigkeiten aufgeführt, die zu kratzfesten, lösungsmittelfesten Lackierungen führten.

Tabelle 1

| Initiator | Reaktivität (m/min) | |
|---|---|---|
| Benzildimethylketal | 5 | (Vergleich) |
| 2-Benzoyl-2-phenyl-4,7-dihydro-1,3-dioxepin | 5 | |
| 2-Benzoyl-2-phenyl-1,3-dioxolan | 2,5 | (Vergleich) |
| 2-Benzoyl-2-phenyl-5,5-dimethyl-1,3-dioxan | 2,5 | (Vergleich) |

Beispiel 7

Ein strahlenhärtbarer Lack, bestehend aus 100 Teilen Bindemittel 2 und 2,5 Teilen der in Tabelle 2 angegebenen Photoinitiatoren, wurde zu einem 50 µm bzw. 250 µm dicken Film auf eine Glasplatte aufgezogen und sofort bei 5 m/min Bandgeschwindigkeit unter einem UV-Brenner gehärtet. Nach 2-stündiger Lagerung wurden die Pendelhärten nach DIN 53 157 bestimmt.

(Siehe Tabelle 2 Seite 7 f.)

Tabelle 2

| Photoinitiator | Pendelhärte (sec) 50 µm | 250 µm | |
|---|---|---|---|
| Benzildimethylketal | 49 | 38 | (Vergleich) |
| 2-Benzoyl-2-phenyl-4,7-dihydro-1,3-dioxepin | 58 | 50 | |
| 2-Benzoyl-2-phenyl-1,3-dioxolan | + | | (Vergleich) |
| 2-Benzoyl-2-phenyl-5,5-dimethyl-1,3-dioxan | + | | (Vergleich) |

+) Lacke nach 2 Passagen nicht kratzfest und leicht klebrig.

Zur Prüfung der Vergilbung wurden die in Tabelle 2 aufgeführten Proben im Falle von Benzildimethylketal und 2-Benzoyl-2-phenyl-4,7-dihydro-1,3-dioxepin halbseitig abgedeckt und anschließend nochmals mit 3 Passagen bei 5 m/min unter einem UV-Brenner belichtet. Die Ergebnisse dieses Vergilbungsversuches sind in Tabelle 3 aufgeführt.

Tabelle 3

| Initiator | |
|---|---|
| Benzildimethylketal | starke Vergilbung |
| 2-Benzoyl-2-phenyl-4,7-dihydro-1,3-dioxepin | schwache Vergilbung |

Beispiel 8

Reaktivitäten verschiedener 2-Benzoyl-2-phenyl-1,3-dioxaalkane bzw. -alkene

Ein strahlenhärtbarer Lack, bestehend aus 100 Teilen Bindemittel 2 und 2,5 Teilen der in Tabelle 4 angegebenen Photoinitiatoren, wurde zu einem 50 µm bzw. 250 µm dicken Film auf eine Glasplatte aufgezogen und sofort bei 5 m/min Bandgeschwindigkeit unter einem UV-Brenner gehärtet.

Tabelle 4

| Initiator | 50 µm | 250 µm |
|---|---|---|
| 2-Benzoyl-2-phenyl-1,3-dioxan | klebrig | klebrig |
| 2-Benzoyl-2-phenyl-1,3-dioxepan | klebfrei nicht kratzfest | klebfrei nicht kratzfest |
| 2-Benzoyl-2-phenyl-4,7-dihydro-1,3-dioxepin | klebfrei kratzfest | klebfrei kratzfest |
| 2-Benzoyl-2-phenyl-1,3-dioxolan | fast klebfrei, nicht kratzfest | fast klebfrei, nicht kratzfest |

**Patentansprüche** (für die Vertragsstaaten : BE, CH, DE, FR, GB, IT, LI, NL)

1. Verbindungen der Formel

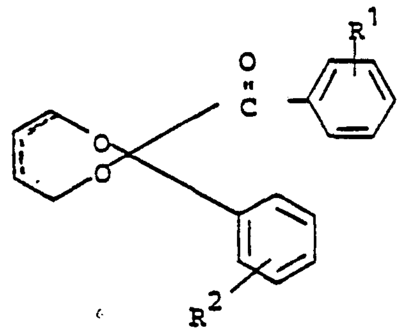

(V)

worin $R^1$ und $R^2$ unabhängig voneinander ein Wasserstoffatom, einen $C_1$-$C_4$-Alkoxy- oder Alkylrest oder ein Halogenatom bedeuten.

2. Verbindungen nach Anspruch 1, dadurch gekennzeichnet, daß der $C_1$-$C_4$-Alkylrest ein Methylrest ist.

3. Verbindungen nach Ansprüchen 1 und 2, dadurch gekennzeichnet, daß das Halogenatom ein Chloratom ist.

4. Verfahren zur Herstellung der Verbindungen nach Ansprüchen 1 bis 3 durch Umsetzung von cis-2-Buten-1,4-diol mit einem Benzil der Formel

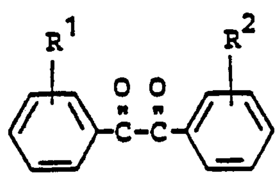

(III)

oder mit einem Benzilmonoketal der Formel

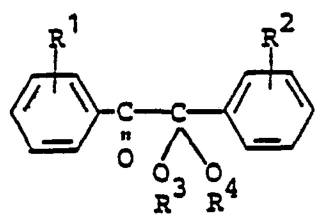

(IV)

wobei

$R^1$ und $R^2$ die oben angegebene Bedeutung besitzen und

$R^3$ und $R^4$ unabhängig voneinander einen $C_1$-$C_4$-Alkylrest bedeuten, im Gegenwart eines sauren Katalysators und gegebenenfalls anschließende Isomerisierung.

5. Verwendung der Verbindungen nach Ansprüchen 1-3 als Photoinitiatoren.

**Patentansprüche** (für den Vertragsstaat AT)

1. Verfahren zur Herstellung von Verbindungen der Formel

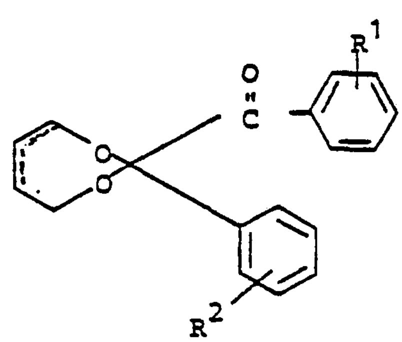

(V)

worin $R^1$ und $R^2$ unabhängig voneinander ein Wasserstoffatom, einen $C_1$-$C_4$-Alkoxy- oder Alkylrest oder ein Halogenatom bedeuten, durch Umsetzung von cis-2-Buten-1,4-diol mit einem Benzil der Formel

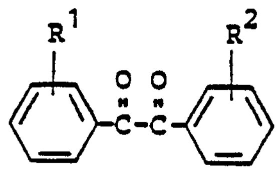

(III)

oder mit einem Benzilmonoketal der Formel

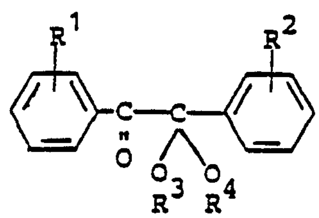

(IV)

wobei

$R^1$ und $R^2$ die oben angegebene Bedeutung besitzen und

$R^3$ und $R^4$ unabhängig voneinander einen $C_1$-$C_4$-Alkylrest bedeuten, in Gegenwart eines sauren Katalysators und gegebenenfalls anschliessende Isomerisierung.

2. Verwendung der Verbindungen nach Anspruch 1 als Photoinitiatoren

**Claims** (for the Contracting States : BE, CH, DE, FR, GB, IT, LI, NL)

1. A compound of the formula

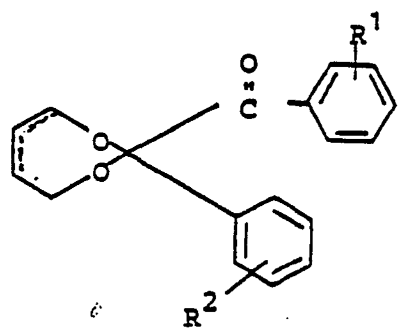

(V)

wherein $R^1$ and $R^2$ are each independently of the other a hydrogen atom, a $C_1$-$C_4$ alkoxy or alkyl radical, or a halogen atom.

2. A compound according to claim 1, wherein the $C_1$-$C_4$ alkyl radical is a methyl radical.

3. A compound according to either claim 1 or claim 2, wherein the halogen atom is a chlorine atom.

4. A process for the preparation of a compound according to any one of claims 1 to 3 by reacting cis-2-butene-1,4-diol with a benzil of the formula

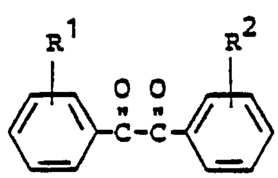

(III)

or with a benzil monoketal of the formula

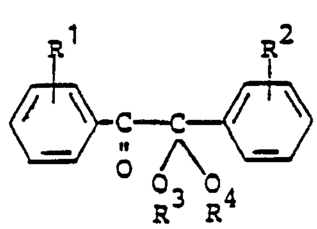

(IV)

wherein

$R^1$ and $R^2$ are as defined above ; and

$R^3$ and $R^4$ are each independently of the other $C_1$-$C_4$ alkyl radical, in the presence of an acid catalyst, followed by subsequent optional isomerisation.

5. Use of a compound according to any one of claims 1 to 3 as photoinitiator.

**Claims** (for the Contracting State AT)

1. A process for the preparation of a compound of the formula

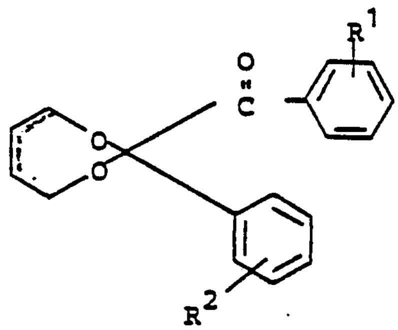

(V)

wherein $R^1$ and $R^2$ are each independently of the other a hydrogen atom, a $C_1$-$C_4$ alkoxy or alkyl radical, or a halogen atom, which comprises reacting cis-2-butene-1,4-diol with a benzil of the formula

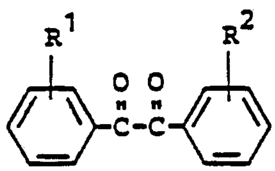

(III)

or with a benzil monoketal of the formula

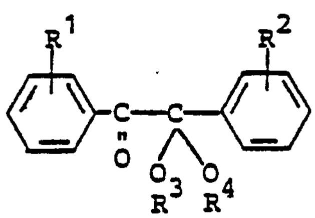

(IV)

wherein

$R^1$ and $R^2$ are as defined above, and

$R^3$ and $R^4$ are each independently of the other a $C_1$-$C_4$ alkyl radical, in the presence of an acid catalyst, followed by subsequent optional isomerisation.

2. Use of a compound according to claim 1 as photoinitiator.

**Revendications** (pour les Etats contractants : BE, CH, DE, FR, GB, IT, LI, NL)

1. Les composés de formule

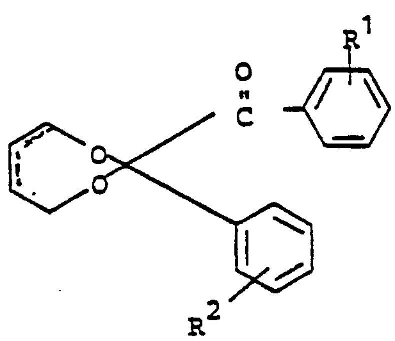

(V)

dans laquelle $R^1$ et $R^2$ représentent chacun, indépendamment l'un de l'autre, un atome d'hydrogène, un alkyle ou un alcoxy en $C_1$ à $C_4$ ou un atome d'halogène.

2. Composés selon la revendication 1 caractérisés en ce que l'alkyle en $C_1$-$C_4$ est le radical méthyle.

3. Composés selon les revendications 1 et 2 caractérisés en ce que l'atome d'halogène est un atome de chlore.

4. Procédé de préparation des composés selon les revendications 1 à 3 par réaction du cis-2-butène-1,4-diol avec un benzile de formule

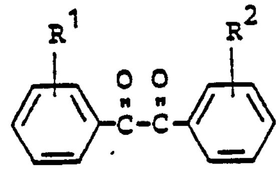

(III)

ou avec un benzile-monoacétal de formule

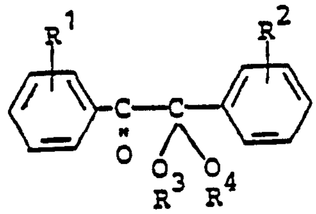

(IV)

$R^1$ et $R^2$ ayant les significations données à la revendication 1 et

$R^3$ et $R^4$ désignant chacun, indépendamment l'un de l'autre, un alkyle en $C_1$-$C_4$, en présence d'un catalyseur acide, réaction qui est le cas échéant suivie d'une isomérisation.

5. L'utilisation comme photo-inducteurs des composés selon les revendications 1 à 3.

**Revendications** (pour l'Etat contractant AT)

1. Procédé de préparation des composés de formule

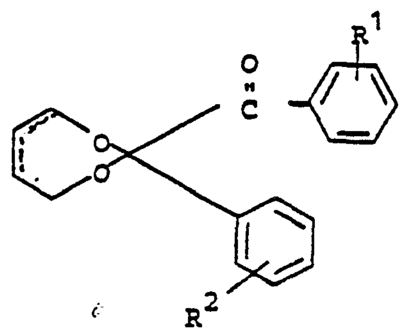

(V)

dans laquelle $R^1$ et $R^2$ désignent chacun, indépendamment l'un de l'autre, un atome d'hydrogène, un alkyle ou un alcoxy en $C_1$ à $C_4$ ou un atome d'halogène, par réaction du cis-2-butène-1,4-diol avec un benzile de formule

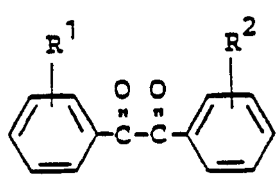

(III)

ou avec un benzile-monoacétal de formule

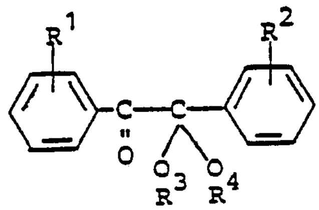

(IV)

$R^3$ et $R^4$ désignant chacun, indépendamment l'un de l'autre, un alkyle en $C_1$-$C_4$, en présence d'un catalyseur acide, réaction qui suivie le cas échéant d'une isomérisation.

2. L'utilisation comme photo-inducteurs des composés obtenus selon la revendication 1.